# FASCICULE DE BREVET EUROPEEN

(11) **EP 0 441 084 B1**
(45) Date de publication et mention de la délivrance du brevet: **20.03.1996**
(21) Numéro de dépôt: 90450018.8
(22) Date de dépôt: 19.12.1990
(51) Int. Cl.: A61B 17/60

(54) **Dispositif d'ostéosynthèse du rachis multifonctionnel**
Multifunktionelle Osteosynthesevorrichtung für die Wirbelsäule
Multifunctional spinal osteosynthesis device

(30) Priorité: 08.02.1990 FR 9001633
(43) Date de publication de la demande: 14.08.1991
(73) Titulaire: STRYKER CORPORATION (a Michigan corporation), Kalamazoo, Michigan 49002 (US); Henry, Patrick, F-92400 Courbevoie (FR)
(72) Inventeur: Vignaud, Jean-Louis, F-33950 Le Piraillan - Lege - Cap Ferret (FR); Henry, Patrick, F-75116 Paris (FR)
(74) Mandataire: Martin, Jean-Jacques

(56) Documents cités:
- EP-A- 0 241 914
- FR-A- 782 462
- FR-A- 2 615 095
- GB-A- 780 652
- US-A- 4 743 260

## Description

La présente invention concerne un dispositif permettant l'ostéosynthèse du rachis par voie postérieure avec des implants, dont une partie est destinée à l'ancrage osseux dans les pédicules vertébraux et l'autre partie permet de recevoir soit une plaque soit une tige pour relier les différentes vis entre elles.

Habituellement, on implante des vis pédiculaires réunies uniquement soit par des plaques, soit par des tiges.

On connaît déjà par FR-A-2 615 095 un dispositif d'osétéosynthèse du rachis selon le préambule de la revendication 1, dans lequel l'élément de liaison est une tige.

Le but de l'invention est de proposer une instrumentation de conception plus simple que ce dispositif connu, permettant un positionnement et un blocage particulièrement précis et sûr, et permettant l'utilisation aussi bien d'une plaque que d'une tige, en vue de faciliter l'ostéosynthèse du rachis, quelles que soient les conditions anatomiques et les déformations en cyphose, en scoliose ou en cyphose scoliose.

A cet effet, l'invention a pour objet un dispositif d'ostéosynthèse du rachis à vis pédiculaires du type comportant une pointe de forme ogivale filetée et une tête filetée, séparées par un corps hexagonal, lesdites vis étant reliées par un élément de liaison, caractérisé en ce qu'il comporte, en outre, une pièce de fixation unique munie d'un trou de passage de ladite tête filetée sur laquelle est vissé un écrou de blocage de l'ensemble pièce de fixation élément de liaison sur ladite tête, l'un des deux organes, pièce de fixation ou élément de liaison, étant conformé sur sa face inférieure de facon à constituer une rainure de réception et blocage en rotation dudit corps hexagonal, ladite pièce en forme de cavalier étant également munie de stries sur sa face en regard de la plaque.

Suivant une application, l'élément de liaison consiste en une tige, et le dispositif est caractérisé en ce que ladite pièce de fixation comporte sur sa face supérieure une rainure dans le fond de laquelle est ménagé ledit trou, dont un des flancs forme un épaulement et dont l'autre flanc délimite une surface concave de réception de ladite tige, ledit écrou de blocage étant muni sur sa face inférieure d'une partie tronconique susceptible de venir en appui, d'une part, contre ledit épaulement et, d'autre part, contre ladite tige et en ce que la face inférieure de ladite pièce de fixation comporte ladite rainure de réception et blocage du corps hexagonal.

Suivant une application, l'élément de liaison consiste en une plaque, et le dispositif est caractérisé en ce que ladite pièce de fixation est une pièce en forme de cavalier chevauchant la face supérieure de ladite plaque munie dudit trou de passage et flanquée de deux saillies latérales parallèles, en ce que ladite plaque comporte des trous oblongs et est munie, sur sa face supérieure, de stries transversales, et, sur sa face inférieure, de ladite rainure de réception et blocage du corps hexagonal.

La figure 1 du dessin annexé est une vue en éclaté d'un mode de réalisation du dispositif de l'invention à la fixation d'une tige.

La figure 2 est une vue en coupe du dispositif de la figure 1 assemblé.

La figure 3 est une vue en éclaté d'un mode de réalisation du dispositif à la fixation d'une plaque.

Le dispositif de l'invention comporte des vis pédiculaires, de type général connu, composées de trois parties : une pointel, un corps 2, une tête 3.

La vis est construite dans des matériaux homologués pour la chirurgie afin d'être bio-compatibles et d'éviter les ruptures de matériel au niveau de sa pénétration dans le corps vertébral.

La pointe de la vis 1 est de forme ogivale filetée et comporte avantageusement une conicité différente entre le fond des filets et leur sommet.

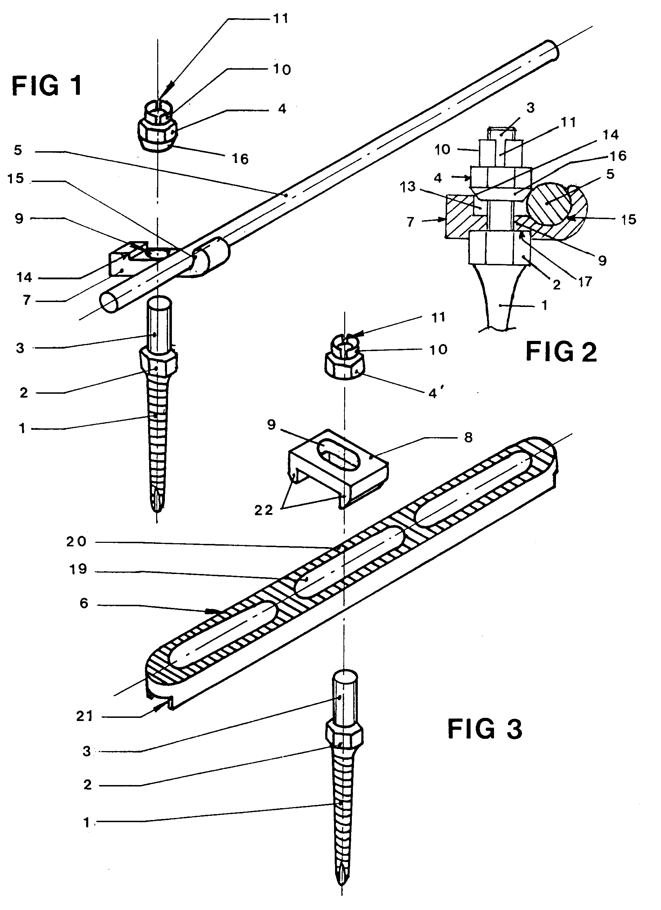

La tête de la vis 3 a une forme cylindrique avec un filetage permettant de recevoir un écrou (4,4') de blocage d'une tige 5 (figure 1) ou d'une plaque 6 (figure 3) par l'intermédiaire d'une pièce auxiliaire unique (7 ou 8) munie dans sa partie centrale d'un trou lisse 9 et enfilée sur la tête 3.

Les écrous (4,4') sont prolongés en partie supérieure par une partie tubulaire cylindrique 10 avec des fentes 11 autorisant un freinage des écrous par un léger rapprochement des parties 10 en regard à l'aide d'une pince avant d'enfiler la tête de vis 3 dans la pièce auxiliaire 7,8.

En outre, des moyens sont ménagés sur l'un des deu organes suivants : la pièce auxiliaire unique ou l'élément de liaison des vis pédiculaires, pour constituer un logement de réception et blocage en rotation du corps 2 des vis par rapport audit élément de liaison.

Dans le mode de réalisation illustré par les figures 1 et 2, la pièce auxiliaire ou intermédiaire unique 7 est interposée entre une tige 5 de section cylindrique d'un diamètre suffisant pour résister aux contraintes et efforts du rachis, la surface lisse ayant l'avantage de permettre un pliage pour, obtenir une certaine courbure, s'adaptant aux déformations ou à l'anatomie du rachis.

Cette pièce 7 comporte, sur sa face supérieure, une rainure centrale 13 dans le fond de laquelle est percé le trou 9. L'un des flancs de la rainure 13 est droit et forme un épaulement 14 et l'autre flanc de la rainure délimite une surface concave 15 servant de logement à la tige 5 à côté du trou 9.

Dans ce mode de réalisation, les écrous 4 sont prolongés en partie inférieure par une partie tronconique 16 susceptible de venir (figure 2) prendre appui, d'une part, sur le rebord de l'épaulement 14 et, d'autre part, sur la tige 5 en place dans son logement 15 lors de la fixation et du blocage de l'ensemble 7, 5 sur une tête de vis 3.

La pièce comporte, sur sa face inférieure, une rainure 17 (figure 2) correspondant au corps 2 et recevant ce dernier en le bloquant en rotation.

Dans le mode de réalisation de la figure 3, la pièce intermédiaire unique 8 a une forme générale de cavalier destiné à chevaucher une plaque lombo-sacrée 18 de section rectangulaire, pré-courbée, comportant de place en place des trous oblongs 19 de réception des têtes de vis 3.

La plaque 18 comporte, sur sa face supérieure, des stries transversales 20 et, sur sa face inférieure, une rainure 215 analogue à la rainure 13 de la pièce 7, et destinée à recevoir et bloquer les corps de vis 2.

La pièce intermédiaire 8 comporte une partie centrale percée d'un trou 9, de préférence oblong pour des facilités d'insertion de la tête de vis 3 et flanquée de deux saillies latérales 22 de guidage et calage de la pièce 8 le long de la face supérieure striée de la plaque 18, la face de la pièce 8 tournée vers les stries 20 étant également striée en correspondance.

Les stries évitent le déplacement, une fois le serrage de l'écrou 4' effectué, de la tête de vis 3 dans le trou oblong 19.

L'écrou 4' est analogue à celui des figures 1 et 2 excepté qu'il ne comporte pas de partie tronconique à sa base.

En modifiant la taille des vis (1,2,3) on peut pratiquer avec le dispositif de l'invention quel que soit sa variante de réalisation, des fixations vertébrales sur toute la hauteur du rachis dorso-lombo-sacré.

## Revendications

1. Dispositif d'ostéosynthèse du rachis à vis pédiculaires du type comportant une pointe (1) de forme ogivale filetée et une tête filetée (3), séparées par un corps hexagonal (2), lesdites vis étant reliées par un élément de liaison (5,6), caractérisé en ce qu'il comporte, en outre, une pièce de fixation unique (7,8) munie d'un trou (9) de passage de ladite tête filetée sur laquelle est vissé un écrou (4,4') de blocage de l'ensemble pièce de fixation (7,8)-élément de liaison (5,6) sur ladite tête (3), l'un des deux organes, pièce de fixation ou élément de liaison, étant conformé sur sa face inférieure de façon à constituer une rainure (17,21) de réception et blocage en rotation dudit corps hexagonal (2).

2. Dispositif suivant la revendication 1,dans lequel l'élément de liaison consiste en une tige (5), caractérisé en ce que ladite pièce de fixation (7) comporte sur sa face supérieure une rainure (13) dans le fond de laquelle est ménagé ledit trou (9), dont un des flancs forme un épaulement (14) et dont l'autre flanc délimite une surface concave (15) de réception de ladite tige (5), ledit écrou de blocage (4) étant muni sur sa face inférieure d'une partie tronconique (16) susceptible de venir en appui, d'une part, contre ledit épaulement (14) et, d'autre part, contre ladite tige (5) et en ce que la face inférieure de ladite pièce de fixation (7) comporte ladite rainure (17) de réception et blocage du corps hexagonal (2).

3. Dispositif suivant la revendication 1, dans lequel l'élément de liaison est une plaque (6) allongée et ajourée de place en place pour le passage des têtes de vis pédiculaires (3), caractérisé en ce que ladite pièce de fixation est une pièce en forme de cavalier (8) chevauchant la face supérieure de ladite plaque (6), munie dudit trou de passage (9) de préférence oblong et flanquéé de deux saillies latérales parallèles (22), en ce que ladite plaque (6) comporte des trous oblongs (19) et est munie, sur sa face supérieure, de stries (20) transversales, et, sur sa face inférieure, de ladite rainure (21) de réception et blocage du corps hexagonal (2), ladite pièce (8) étant également munie de stries sur sa face tournée vers ladite plaque (6).

4. Dispositif suivant l'une des revendications 1 à 3, caractérisé en ce que ledit écrou (4,4') est prolongé sur sa face supérieure par une partie tubulaire cylindrique (10) fendue diamétralement (11) à des fins de freinage de l'écrou.

## Claims

1. Spinal osteosynthesis device with pedicle screws of the type comprising a threaded point (1) of ogival shape and a threaded head (3) which are separated by a hexagonal body (2), the said screws being connected by a joining element (5, 6), characterized in that it additionally comprises a single fixation piece (7, 8) equipped with a hole (9) for passage of the said threaded head on which a nut (4, 4') is screwed for locking the fixation piece (7, 8)/joining element (5, 6) assembly on the said head (3), one of the two members - fixation piece or joining element - being designed on its lower surface in such a way as to constitute a groove (17, 21) for receiving the said hexagonal body (2) and locking it in terms of rotation.

2. Device according to Claim 1, in which the joining element consists of a rod (5), characterized in that the said fixation piece (7) comprises, on its upper surface, a groove (13) in the bottom of which the said hole (9) is formed, one of the flanks thereof forming a shoulder (14) and the other flank delimiting a concave surface (15) for receiving the said rod (5), the said locking nut (4) being equipped on its lower surface with a frustoconical portion (16) capable of bearing, on the one hand, against the said shoulder (14) and, on the other hand, against the said rod (5), and in that the lower face of the said fixation piece (7) comprises the said groove (17) for receiving and locking the hexagonal body (2).

3. Device according to Claim 1, in which the joining element is an elongate plate (6) which is open-worked in places for the passage of the pedicle screw heads (3), characterized in that the said fixation piece is a stirrup-shaped piece (8) which straddles the upper face of the said plate (6), is equipped with the said passage hole (9), which is preferably oblong, and is flanked by two parallel lateral projections (22), and in that the said plate (6) comprises oblong holes (19) and is equipped, on its upper face, with transverse striations (20), and, on its lower face, with the said groove (21) for receiving and locking the hexagonal body (2), the said piece (8) likewise being equipped with striations on its face directed towards the said plate (6).

4. Device according to one of Claims 1 to 3, characterized in that the said nut (4, 4') is continued on its upper face via a cylindrical tubular portion (10) which is slotted diametrally (11) for the purpose of braking the nut.

## Patentansprüche

1. Vorrichtung für die Osteosynthese der Wirbelsäule, mit Stielschrauben des Typs, der eine Spitze (1) in Form eines mit Gewinde versehenen Spitzkegels und einen mit Gewinde versehenen Kopf (3) aufweist, getrennt durch einen sechseckigen Körper (2), wobei die Schrauben durch ein Verbindungselement (5, 6) verbunden sind, dadurch gekennzeichnet, daß sie weiterhin ein eindeutiges Fixierstück (7, 8) aufweist, versehen mit einem Loch (9) für den Durchlaß des mit Gewinde versehenen Kopfes, auf den eine Mutter (4, 4') zum Blockieren der Anordnung Fixierstück (7, 8) - Verbindungselemente (5, 6) auf dem Kopf (3)aufgeschraubt ist, wobei eines der beiden Elemente, Fixierstück oder Verbindungselement, auf seiner unteren Fläche derart ausgebildet ist, daß eine Nut (17, 21) zum Aufnehmen und Drehimmobilisieren des sechseckigen Körpers (2) gebildet wird.

2. Vorrichtung nach Anspruch 1, bei der das Verbindungselement aus einer Stange (5) besteht, dadurch gekennzeichnet, daß das Fixierstück (7) auf seiner oberen Fläche eine Nut (13) aufweist, in deren Boden das Loch (9) ausgebildet ist, wobei eine ihrer Seitenwände eine Schulter (14) bildet und ihre andere Seitenwand eine konkave Fläche (15) zum Aufnehmen der Stange (5) begrenzt, wobei die Blockiermutter (4) auf ihrer unteren Fläche mit einem kegelstumpfartigen Abschnitt (16) versehen ist, der einerseits gegen die Schulter (14) und andererseits gegen die Stange (5) in Anlage kommen kann, und daß die untere Fläche des Fixierstückes (7) die Nut (17) zum Aufnehmen und Blockieren des sechseckigen Körpers (2) aufweist.

3. Vorrichtung nach Anspruch 1, bei der das Verbindungselement eine längliche und stellenweise durchbrochene Platte (6) für den Durchlaß der Köpfe (3) der Stielschrauben ist, dadurch gekennzeichnet, daß das Fixierstück ein Stück in Form eines Reiters (8) ist, der rittlings auf der oberen Fläche der Platte (6) sitzt, mit dem Durchlaßloch (9), bevorzugt länglich, versehen und durch zwei seitliche, parallele Vorsprünge (22) begrenzt, und daß die Platte (6) Langlöcher (19) aufweist und auf ihrer Oberfläche mit querverlaufenden Rillen (20) und auf ihrer unteren Fläche mit der Nut (21) für die Aufnahme und Blockierung des hexagonalen Körpers (2) versehen ist, wobei das Stück (8) gleichermaßen mit Rillen auf seiner Fläche versehen ist, die der Platte (6) zugewandt ist.

4. Vorrichtung nach einem der Ansprüche 1 bis 3, dadurch gekennzeichnet, daß die Mutter (4, 4') auf ihrer oberen Fläche durch einen rohrförmigen, zylindrischen Abschnitt (10) verlängert ist, der diametral (11) in Endstücke zum Sichern der Mutter geschlitzt ist.
